# EUROPÄISCHE PATENTSCHRIFT

(11) **EP 2 198 893 B1**
(45) Veröffentlichungstag und Bekanntmachung des Hinweises auf die Patenterteilung: **29.10.2014**
(21) Anmeldenummer: 09014542.6
(22) Anmeldetag: 23.11.2009
(51) Int. Cl.: A61L 27/16, A61L 24/06

(54) **Sporozide Zusammensetzungen und deren Verwendung**
Sporicidal compounds and use of same
Compositions sporozoïdes et leur utilisation

(30) Priorität: 18.12.2008 DE 102008063524; 20.01.2009 DE 102009005534
(43) Veröffentlichungstag der Anmeldung: 23.06.2010
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 64354 Reinheim (DE)
(74) Vertreter: Kühn, Hans-Christian

(56) Entgegenhaltungen:
- EP-A1- 1 438 976
- US-A- 4 537 940
- US-A1- 2004 157 952
- US-A1- 2005 008 528
- US-A1- 2006 052 471
- US-A1- 2008 293 845

## Beschreibung

Gegenstand der Erfindung sind sporozide Zusammensetzungen und deren Verwendung, insbesondere als oder zur Herstellung von Knochenzementpasten.

PMMA-Knochenzemente (Polymethylmethacrylat-Knochenzemente) sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Der Grundaufbau der PMMA-Knochenzemente ist seit dem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einem darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. PMMA-Knochenzemente sind Medizinprodukte der Klasse IIb bzw. bei Zusatz von Antibiotika der Klasse III. Es ist daher erforderlich, dass die Zemente nur im sterilen Zustand in doppelter steriler Verpackung in den Verkehr gebracht werden dürfen, um die Sicherheit der Patienten zu gewährleisten. Die Pulverkomponente wird bei den meisten PMMA-Knochenzementen durch Ethylenoxid-Einwirkung sterilisiert. Daneben ist auch die Sterilisation der Pulverkomponente durch Gammabestrahlung üblich. Die Sterilität der Monomerflüssigkeit wird durch Sterilfiltration und nachfolgender aseptischer Abfüllung erreicht. Das für die Herstellung der Monomerflüssigkeit eingesetzte Methylmethacrylat ist auf Grund seiner lipophilen und damit denaturierenden Eigenschaften für die meisten vegetativen mikrobiellen Lebensformen biozid. In wasserfreiem Methylmethacrylat können Mikroorganismen nicht existieren. Mikrobielles Leben ist immer an die Verfügbarkeit von Wasser gebunden. Neben den vegetativen Formen der Mikroorganismen gibt es auch generative Formen, wie Endosporen. Sie stellen generative Überdauerungsformen von Mikroorganismen dar und werden von Gram-positiven Bakterien, besonders der Gattungen *Bacillus* und *Clostridium* gebildet, um ungünstige Lebensbedingungen überdauern zu können. Endosporen haben im Ruhezustand keinen aktiven Stoffwechsel und besitzen eine mehrschichtige Sporenhülle, die den Sporen-Kern vor Einwirkung von Chemikalien und anderen Umwelteinflüssen weitgehend schützt. Dadurch sind Endosporen extrem widerstandsfähig gegenüber der Einwirkung von Hitze und von Chemikalien (Borick, P. M.: Chemical sterilizers. Adv. Appl. Microbiol. 10 (1968) 291-312; Gould, G. W.: Recent advances in the understanding of resistance and dormacy in bacterial spores. J. Appl. Bacteriol. 42 (1977) 297-309; Gould, G. W.: Mechanisms of resistance and dormancy. p. 173-209. In Hurst, A. and Gould, G. W. (ed.), The bacterial spore. vol. 2 Academic Press, Inc. New York, 1983). Endosporen werden auf Grund ihrer hohen Widerstandsfähigkeit als Bioindikatoren für die Validierung und die Wirksamkeitskontrolle von Sterilisationsprozessen eingesetzt. Es wird dabei davon ausgegangen, dass eine Inaktivierung der Sporen eine Abtötung aller vegetativen mikrobiellen Lebensformen abbildet. Endosporen von Gram-positiven Bakterien gehören der internationalen Resistenzstufe III an. Zu den Resistenzstufen I gehören nichtsporenbildende Bakterien und vegetative Formen von Sporenbildnern und zur Resistenzstufe II zählen Sporen, die in strömenden Wasserdampf bei 105 °C innerhalb weniger Minuten abgetötet werden. Bei einer Sterilisation müssen nach DAB 2008 alle Mikroorganismen der Resistenzstufen I-III abgetötet bzw. irreversibel inaktiviert werden.

Neben physikalischen Sterilisationsprozessen wie Gammabestrahlung, Elektronenbestrahlung, UV-Bestrahlung, Hitzesterilisation und Autoklavieren mit gespanntem Dampf werden auch chemische Agenzien zur Sterilisation eingesetzt. Dazu gehören Ethylenoxid, Formaldehyd, Glutardialdehyd, o-Phthaldialdehyd, Hypochlorit, Chlordioxid, Jod, Peressigsäure und Wasserstoffperoxid. Ethylendioxid wirkt nur in Gegenwart von Feuchtigkeit sporozid. Ähnlich ist die Wirksamkeit der Aldehyde. Sie werden üblicherweise als wässrige Lösungen verwendet oder im Fall von Formaldehyd im gasförmigen Zustand. Sehr wirksam sind auch die auf Chlor basierenden Agenzien. Der Nachteil ist dabei, dass Chlor enthaltende Folgeprodukte nach der Keimabtötung verbleiben. Peressigsäure und Wasserstoffperoxid werden als stark oxidierende Agenzien ebenfalls in Form von wässrigen Lösungen verwendet. Daneben ist auch die Sterilisation mit gasförmigem Wasserstoffperoxid möglich. Beide Substanzen wirken bakterizid, fungizid, viruzid und sporozid (Russell, A. D.: Bacterial spores and chemical sporocidal agents. Clin. Microbiol. Rev. Vol. 3, No. 2 (1990) 99-119). Wasserstoffperoxid ist eine relativ instabile Verbindung, die in wässriger Lösung langsam zu Sauerstoff und Wasser zerfällt. Es hat keine mutagenen/cancerogenen Eigenschaften. Generell hängt der Erfolg der chemischen Sterilisationsprozesse von der Konzentration des chemischen Agens, der Einwirkungszeit und der Wasseraktivität ab.

Eine Weiterentwicklung der Polymethylmethacrylat-Knochenzemente stellen pastenförmige auf Polymethylmethacrylat basierende Zemente dar. Sie enthalten ebenfalls neben Polymethylmethacrylat oder Polymethylmethacrylat-Copolymeren, einen Röntgenopaker und Methylmethacrylat als Monomer und gegebenenfalls weitere Methacrylatmonomere. Wasser ist nur in Spuren vorhanden. Auf Grund der lipophilen, denaturierenden Eigenschaften des Methylmethacrylats und auf Grund der weitgehenden Wasserfreiheit können vegetative Lebensformen von Mikroorganismen in den Zementpasten nicht überleben. Ruhende Endosporen besitzen die Fähigkeit, längere Zeit ohne Inaktivierung in Methacrylatmonomeren zu überdauern. Daher ist es notwendig, eine sichere Abtötung/Inaktivierung aller mikrobiellen Lebensformen, einschließlich von ruhenden Endosporen, in den Zementpasten zu erreichen. Zementpasten können auf Grund der enthaltenden Methacrylatmonomere nicht durch Hitze- und Dampfeinwirkung sowie durch Gamma- und Röntgenbestrahlung sterilisiert werden, weil diese Sterilisationsmethoden eine radikalische Polymerisation der Monomere auslösen könnten oder im Fall der Dampfsterilisation eine Hydrolyse verursachen würden. Ebenfalls ist die Verwendung von Ethylenoxid nicht möglich. Ethylenoxid kann nicht in geschlossene, diffusionsdichte Folienbeutel eindringen. Eine Sterilfiltration kann auf Grund der hohen Viskosität der Zementpasten und der in den Zementpasten enthaltenen Opaker- und Füllstoffpartikel nicht durchgeführt werden. Alternativ ist noch die aseptische Herstellung möglich. Diese ist jedoch extrem kostenintensiv.

Die Aufgabe der Erfindung bestand deshalb darin, eine kostengünstige, einfache Möglichkeit zu finden, um Monomere oder Monomerlösungen so zu modifizieren, dass diese mindestens temporäre sporozide Eigenschaften besitzen und dass dadurch die Monomere oder Monomerlösungen autosteril sind, um damit insbesondere sterile Zementpasten herstellen zu können. Wichtig ist weiterhin, dass die so modifizierten Monomere oder Monomergemische hinreichend stabil sind und problemlos mit konventionellen radikalischen Initiatorsystemen polymerisiert werden können.

Der Erfindung liegt die überraschende Beobachtung zu Grunde, dass geringe Mengen wässrige Wasserstoffperoxid-Lösung mit Methylmethacrylat oder anderen Methacrylatmonomeren homogen vermischt werden können und dass diese Lösungen für einige Tage sporozide Eigenschaften besitzen. Ebenso ist es möglich, gasförmiges Wasserstoffperoxid in Methylmethacrylat oder anderen Methacrylatmonomeren homogen zu lösen. Durch Zusatz eines in Methylmethacrylat löslichen Stabilisators bleibt das Methylmethacrylat stabil. Der Stabilisator wird dabei gegenüber dem Wasserstoffperoxid im Überschuss eingesetzt. Überraschend war dabei, dass die Inaktivierung von Endosporen trotz der Anwesenheit von Stabilisatoren erfolgt. Der Stabilisator wird letztlich unter Verbrauch des Wasserstoffperoxids oxidiert. Als Nebenprodukt fällt neben dem oxidierten Stabilisator nur das nichttoxische Wasser in Spuren an. Der wesentliche Vorteil dieses autosterilen Monomer/Monomergemisch besteht darin, dass die damit hergestellten Zementpasten unmittelbar nach der Herstellung über einen Zeitraum von mehreren Tagen eine sporozide Wirksamkeit haben. Dadurch ist es möglich, die Zementpasten in geeignete diffusionsdichte Verbundfolienbeutel einzusiegeln. Die eingesiegelte Zementpaste inaktiviert an der Innenseite der Verbundfolienbeutel anhaftende Mikroorganismen irreversibel durch Oxidation. Das bedeutet, die Zementpaste und die Innenseite des Schlauchbeutels wird durch Einwirkung des erfindungsgemäßen Monomers/Monomergemischs sterilisiert. Es wurde in eingehenden Versuchen nachgewiesen, dass eine Reduktion von Endosporen um 6 Log-Stufen sicher erreicht wird. Überraschend war weiterhin, dass die erfindungsgemäßen Monomergemische auch bei extrem niedrigen Wasserstoffperoxid-Konzentrationen von 50 ppm eine sporozide Wirkung zeigten.

Die Aufgabe der Erfindung wird durch Zusammensetzungen nach Anspruch 1 gelöst. Weitere bevorzugte Ausgestaltungen sind den Ansprüchen 2 - 6 zu entnehmen. Die Erfindung betrifft auch die Verwendung erfindungsgemäßer Zusammensetzungen nach Anspruch 7.

### Komponente A

Unter dem Begriff Methacrylatmonomere werden allgemein Ester der Methacrylsäure mit aliphatischen, cycloaliphatischen und aromatischen Alkoholen verstanden, wobei die Ester auch mit Dialkoholen, Trialkoholen oder auch anderen Polyalkoholen gebildet sein können. Der wesentlichste Vertreter dieser Monomere ist Methylmethacrylat. Unter dem Begriff Methacrylatmonomer wird auch Methacrylsäureamid und einfach N-substituierte oder zweifach N,N-substituierte Methacrylsäureamide verstanden.

### Komponente B

Wasserstoffperoxid wird den Zusammensetzungen zweckmäßigerweise als 30%ige Lösung zugesetzt. Wasserstoffperoxid kann *in situ* aus geeigneten Wasserstoffperoxid-Addukten freigesetzt werden. Beispielsweise ist es möglich, aus Erdalkaliperoxiden oder Alkaliperoxiden durch Einwirkung von in Methacrylatmonomeren löslichen Säuren *in situ* Wasserstoffperoxid freizusetzen. Als organische Säuren kommen neben der 2-Ethylhexansäure und der Methacrylsäure alle organischen Säuren in Betracht, die in Methacrylatmonomeren löslich sind und deren Alkali- und Erdalkalisalze ebenfalls in Methacrylatmonomeren löslich sind. Im Rahmen der Erfindung ist ebenfalls der Zusatz von Derivaten des Wasserstoffperoxids, wie Peressigsäure, Perameisensäure, Perpropionsäure, Perphthalsäure, Perbenzoesäure und 3-Chlorperbenzoesäure. Dabei ist es zur Erreichung einer optimalen Sporozidie vorteilhaft, dem Monomer/Monomergemisch Spuren von Wasser hinzuzufügen. Weitere, geeignete Wasserstoff abgebende Substanzen oder Substanzgemische sind dem Fachmann bekannt. Bevorzugte derartige Substanzen sind Wasserstoffperoxid-Harnstoff-Addukt und Natriumcarbonat-Wasserstoffperoxid-Addukt; bevorzugte Gemische die Kombinationen Natriumcarbonat-Wasserstoffperoxid-Addukt/2-Ethylhexansäure, Calciumperoxid/2-Ethylhexansäure, sowie Magnesiumperoxid/2-Ethylhexansäure.

### Komponente C

Als radikalische Stabilisatoren kommen sämtliche stabilisierenden Verbindungen in Frage, die in der Polymerchemie als radikalische Stabilisatoren eingesetzt werden. Sie sollten als Reduktionsmittel wirksam sein und mit Radikalen reagieren können. Die Stabilisatoren müssen in der Lage sein, Sauerstoff zu binden. Bevorzugte Beispiele solcher Stabilisatoren sind Hydrochinon, Hydrochinonmonomethylether, Palmitoylascorbinsäure, 2,6-Di-t-butyl-4-methylphenol (BHT) und Alkylgallate.

Wesentlich für die Erfindung ist, dass das Stoffmengenverhältnis von radikalischem Stabilisator zu Wasserstoffperoxid größer gleich 1 zu 1 ist. Dadurch wird gewährleistet, dass die beim Zerfall des Wasserstoffperoxids frei werdenden Radikale sicher abgefangen werden und eine Spontanpolymerisation des Monomers/Monomergemischs verhindert wird. Der radikalische Stabilisator sollte auch deshalb immer in einem mindestens äquimolaren Stoffmengenverhältnis zum Wasserstoffperoxid vorliegen, damit der bei Zerfall des Wasserstoffperoxids frei werdende Sauerstoff gebunden wird und sich keine Sauerstoffblasen im Monomer/Monomergemisch bilden können. Das Wasserstoffperoxid oder das freigesetzte Wasserstoffperoxid und der radikalische Stabilisator sind in dem Monomer bzw. Monomerengemisch homogen gelöst.

Die Konzentration des Wasserstoffperoxid im Monomer/Monomerengemisch liegt bevorzugt bei 20 ppm bis 0,5 %, besonders bevorzugt bis 0,01 %, bezogen auf die Masse des Monomers oder Monomerengemischs.

Die Zusammensetzungen der Erfindung werden bevorzugt zur Herstellung von Einkomponenten-Knochenzementpasten, von Zweikomponenten-Knochenzementpasten oder von Monomerlösungen für aus Zementpulver und Monomerflüssigkeit aufgebauten Polymethylmethacrylatknochenzementen verwendet.

Eine besonders bevorzugte Zusammensetzung enthält die Komponenten
**A1** ein niedermolekulares, flüssiges Methacrylat,
**A2** ein lineares oder verzweigtes Polymethylmethacrylat oder ein lineares oder verzweigtes Methylmethacrylat-Copolymer,
**A3** ein vernetztes Polymethylmethacrylat oder ein vernetztes Methylmethacrylat-Copolymer,
**B1** Wasserstoffperoxid und/oder
**B2** eine Wasserstoffperoxid freisetzende Substanz oder ein Wasserstoffperoxid freisetzendes Substanzgemisch,
**C** mindestens einen radikalischen Stabilisator und
**D** mindestens eine Komponente eines radikalischen Initiatorsystems.

Sie kann als Basis einer sporoziden Zementpaste, die zusätzlich pharmazeutische Wirkstoffe und Wachstumsfaktoren enthalten kann, wie z.B. Antibiotika, Antiphlogistika, Cytostatika, Immunmodulatoren, Bisphosphonate, Steroidhormone, BMP (Bone morphogenetic proteins). Weiterhin können Zirkoniumdioxid, Bariumsulfat, Jod-organische Röntgenopaker, Tantal und Wolfram als Röntgenopaker in die Zementpaste inkorporiert werden. Daneben ist es auch möglich, superparamagnetische Nanopartikel und ferromagnetische Partikel in der Zementpaste zu suspendieren. Mit diesen Partikeln kann die Zementpaste durch Einwirkung von magnetischen Wechselfeldern erwärmt werden, wobei über den Zerfall geeigneter thermisch zerfallender Initiatoren die radikalische Polymerisation und damit die Aushärtung der Zementpaste induziert werden kann.

Die Erfindung wird durch die folgenden Beispiele näher erläutert, ohne jedoch die Erfindung darauf zu beschränken. Teile und Prozentangaben beziehen sich wie in der übrigen Beschreibung auf das Gewicht, sofern nicht anders angegeben.

### 1. Herstellung und Prüfung von sporoziden Methylmethacrylat-Lösungen

Es wurden in jeweils 50,0 g Methylmethacrylat 30 mg 2,6-Di-t-butyl-4-methylphenol und 50 µl, 25 µl, 10 µl und 5 µl 30 %ige Wasserstoffperoxid-Lösung gelöst. In die Lösungen wurden jeweils 5 Sporenstreifen (Fa. Sterix), die 3 x 10⁶ Sporen des *bacillus atropheus* (*bacillus subtilis*) enthielten, eingebracht. Als Kontrolle wurde eine Lösung von 60,0 g Methylmethacrylat und 30 mg 2,6-Di-t-butyl-4-methyl-phenol eingesetzt, die kein Wasserstoffperoxid enthielt. Die Lösungen wurden bei 37 °C drei Tage inkubiert. Danach wurden die Sporenstreifen entnommen und bei Raumtemperatur getrocknet. Zur Bestimmung des Gehaltes an vitalen Sporen wurden die Sporen aus den Sporenstreifen ausgewaschen, bebrütet und anschließend quantifiziert.

| Zugesetztes Volumen an 30 %iger Wasserstoffperoxid-Lösung | Vitale Sporen pro Sporenstreifen (Mittelwert von 5 Sporenstreifen) |
|---|---|
| - | 3 x 10⁻⁵ |
| 50 µl | 0 |
| 25 µl | 0 |
| 10 µl | 0 |
| 5 µl | 0 |

### 2. Herstellung und Prüfung von sporoziden Zweikomponenten-Zementpasten

Es wurden Zweikomponenten-Pastenzemente mit den Komponenten A und B hergestellt. Die Komponenten A und B wurden jeweils durch einfaches Vermischen der Rohstoffe bei Raumtemperatur hergestellt.

| Zusammensetzung | |
|---|---|
| Komponente A | Komponente B |
| 3,0 g Zirkoniumdioxid | 3,0 g Zirkoniumdioxid |
| 15,0 g Methylmethacrylat | 15,0 g Methylmethacrylat |
| 10,0 g Polymethylmethacrylat | 10,0 g Polymethylmethacrylat |
| 9,0 g vernetztes Polymethylmethacrylat | 9,0 g vernetztes Polymethylmethacrylat |
| 1,0 g 1-Cyclohexyl-5-ethyl-barbitursäure | 0,5 g Trioctylmethylammoniumchlorid |
| 30 mg 2,6-Di-t-butyl-4-methyl-phenol | 100 ppm Kupfer(II)octoat |
| | 30 mg 2,6-Di-t-butyl-4-methyl-phenol |

Die Komponente A wurde mit 10 µl bzw. 5 µl 30 % Wasserstoffperoxid-Lösung vermischt. Die Komponente B wurde ebenfalls mit 10 µl bzw. 5 µl 30%ige Wasserstoffperoxid-Lösung vermischt. In die homogenen Zementpasten wurden jeweils 5 Sporenstreifen (Fa. Sterix), die 3 x 10⁶ Sporen des *bacillus atropheus* (*bacillus subtilis*) enthielten, eingebracht. Als Kontrollen wurden die Zementpasten A und B ohne Zusatz von Wasserstoffperoxid mitgeführt. Es wurde drei Tage bei 37 °C inkubiert. Danach wurden die Sporenstreifen entnommen und bei Raumtemperatur getrocknet. Zur Bestimmung des Gehaltes an vitalen Sporen wurden die Sporen aus den Sporenstreifen ausgewaschen, bebrütet und anschließend quantifiziert.

| Zugesetztes Volumen an 30 %iger Wasserstoffperoxid-Lösung | Vitale Sporen pro Sporenstreifen (Mittelwert von 5 Sporenstreifen) | |
|---|---|---|
| | Paste A | Paste B |
| - | 3 x 10⁵ | 3 x 10⁵ |
| 10 µl | 0 | 0 |
| 5 µl | 0 | 0 |

Es wurden im Folgenden die mechanischen Eigenschaften von Zement, der aus den mit Wasserstoffperoxid modifizierten Pasten A und B hergestellt wurde, bestimmt. Dazu wurden die mit Wasserstoffperoxid modifizierten Pasten A und B im Gewichtsverhältnis 1:1 miteinander vermischt und anschließend in rechteckige Formen mit einer Höhe 3,2 mm eingebracht. Nach erfolgter Aushärtung wurden für die Prüfung der 4-Punkt-Biegefestigkeit und des Biegemoduls Streifen mit einer Länge von 75 mm und einer Breite von 10 mm gesägt. Zur Bestimmung der Dynstat-Biegefestigkeit und der Schlagzähigkeit wurden zusätzlich Prüfkörper mit einer Länge von 20 mm und einer Breite von 10 mm gesägt. Die Prüfung der 4-Punkt-Biegung und des Biegemoduls erfolgte nach 48 Stunden Lagerung der Prüfkörper in Wasser bei 37 °C mit einer Zwick-Universal-Prüfmaschine. Die Dynstat-Biegefestigkeit und die Dynstat-Schlagzähigkeit wurden mit einer Dynstat-Prüfmaschine nach 24 Stunden Lagerung der Prüfkörper an Luft bei 23 °C ermittelt.

| Rezeptur | 4-Punktbiegefestig-keit [MPa] | Biegemodul [MPa] | Dynstat-Biegefestigkeit [MPa] | Dynstat-Schlagzähigkeit [kN/m²] |
|---|---|---|---|---|
| Paste A+ B mit jeweils 10 µl Wasserstoffperoxid-Lösung | 67,8 ± 1,5 | 2698 ± 39 | 86,1 ± 5,7 | 4,07 ± 0,28 |
| Paste A+ B mit jeweils 5 µl Wasserstoffperoxid-Lösung | 65,8 ± 1,0 | 2494 ± 27 | 90,4 ± 5,4 | 3,17 ± 0,06 |

### 3. Herstellung einer sporoziden Einkomponenten-Zementpaste

Es wurde eine Einkomponenten-Zementpaste durch einfaches Vermischen der nachfolgend aufgeführten Rohstoffe bei Raumtemperatur hergestellt. Es entstand eine bräunliche, zähe Paste, die problemlos geformt und modelliert werden konnte.

| Zusammensetzung der Zementpaste |
|---|
| 2,0 g Zirkoniumdioxid |
| 1,0 g Magnetitpartikel |
| 15,0 g Methylmethacrylat |
| 10,0 g Polymethylmethacrylat |
| 9,0 g vernetztes Polymethylmethacrylat |
| 50 mg α,α-Azobis(isobutyronitril) (AIBN) |
| 30 mg 2,6-Di-t-butyl-4-methyl-phenol |
| 10 µl 30%ige Wasserstoffperoxid-Lösung |

Die Aushärtung der Zementpaste erfolgte mit Hilfe einer bei konventionellen Induktionsherden entlehnten Induktionsheizung (Spule mit Steuerungselektronik, Frequenz 25 kHz). Die Polymerisation setzte nach ca. 60 Sekunden ein und führte zu stabilen Formkörpern.

### 4. Herstellung einer sporoziden Einkomponenten-Zementpaste

Es wurde eine Einkomponenten-Zementpaste durch einfaches Vermischen der nachfolgend aufgeführten Rohstoffe bei Raumtemperatur hergestellt. Es entstand eine bräunliche, zähe Paste, die problemlos geformt und modelliert werden konnte.

| Zusammensetzung der Zementpaste |
|---|
| 2,0 g Zirkoniumdioxid |
| 1,0 g Magnetitpartikel |
| 15,0 g Methylmethacrylat |
| 10,0 g Polymethylmethacrylat |
| 9,0 g vernetztes Polymethylmethacrylat |
| 50 mg α,α-Azobis(isobutyronitril) (AIBN) |
| 30 mg 2,6-Di-t-butyl-4-methyl-phenol |
| 50 mg Harnstoff-Wasserstoffperoxid-Addukt |
| 50 µl Wasser |

Die Aushärtung der Zementpaste erfolgte mit Hilfe einer bei konventionellen Induktionsherden entlehnten Induktionsheizung (Spule mit Steuerungselektronik, Frequenz 25 kHz). Die Polymerisation setzte nach 60-70 Sekunden ein und führte zu stabilen Formkörpern.

## Patentansprüche

1. Sporozide Zusammensetzung enthaltend
**A** ein Methacrylatmonomer oder ein Gemisch von Methacrylatmonomeren,
**B1** Wasserstoffperoxid und/oder
**B2** eine Wasserstoffperoxid freisetzende Substanz oder ein Wasserstoffperoxid freisetzendes Substanzgemisch und
**C** mindestens einen radikalischen Stabilisator,
wobei die Komponente **B1** und das aus der Komponente **B2** freigesetzte Wasserstoffperoxid sowie die Komponente **C** in der Komponente **A** homogen gelöst sind, und
das Stoffmengenverhältnis von **C** zu **(B1+B2)** größer oder gleich 1 zu 1 [mol/mol] ist.

2. Zusammensetzung nach Anspruch 1, wobei die Wasserstoffperoxid freisetzenden Substanzen aus einer Gruppe **B21** bestehend aus
• Wasserstoffperoxid-Harnstoff-Addukt und
• Natriumcarbonat-Wasserstoffperoxid-Addukt
und die Wasserstoffperoxid freisetzenden Substanzgemische aus einer Gruppe **B22** bestehend aus
• Natriumcarbonat-Wasserstoffperoxid-Addukt/2-Ethylhexansäure,
• Calciumperoxid/2-Ethylhexansäure und
• Magnesiumperoxid/2-Ethylhexansäure
ausgewählt sind.

3. Zusammensetzung nach mindestens einem der vorstehenden Ansprüche, wobei Wasserstoffperoxid in einer Menge von 20 ppm bis 0,5 Gew %, bezogen auf das Monomer oder Monomerengemisch vorliegt.

4. Zusammensetzung nach Anspruch 3, wobei die Menge 20 ppm bis 0,01 Gew% beträgt.

5. Zusammensetzung nach mindestens einem der vorstehenden Ansprüche, wobei der Stabilisator C der Gruppe bestehend aus Hydrochinon, Hydrochinonmonomethylether, Alkylgallate, Palmitoylascorbinsäure und 2,6-Di-t-butyl-4-methylphenol angehört.

6. Zusammensetzung nach einem der vorstehenden Ansprüche enthaltend
**A1** ein niedermolekulares, flüssiges Methacrylat,
**A2** ein lineares oder verzweigtes Polymethylmethacrylat oder ein lineares oder verzweigtes Methylmethacrylat-Copolymer,
**A3** ein vernetztes Polymethylmethacrylat oder ein vernetztes Methylmethacrylat-Copolymer,
**B1** Wasserstoffperoxid und/oder
**B2** eine Wasserstoffperoxid freisetzende Substanz oder ein Wasserstoffperoxid freisetzendes Substanzgemisch und
**C** mindestens einen radikalischen Stabilisator,
**D** mindestens eine Komponente eines radikalischen Initiatorsystems.

7. Verwendung einer Zusammensetzung nach mindestens einem der Ansprüche 1-5 zur Herstellung von
• Einkomponenten-Knochenzementpasten,
• Zweikomponenten-Knochenzementpasten oder
• Monomerlösungen für aus Zementpulver und Monomerflüssigkeit aufgebauten Polymethylmethacylatknochenzementen.

## Claims

1. Sporicidal composition comprising
**A** a methacrylate monomer or a mixture of methacrylate monomers;
**B1** hydrogen peroxide and/or
**B2** a hydrogen peroxide-releasing substance or a hydrogen peroxide-releasing mixture of substances; and
**C** at least one radical stabiliser;
whereby component **B1** and the hydrogen peroxide released from component **B2** as well as component **C** are dissolved homogeneously in component **A** and the molar ratio of **C** to **(B1+B2)** is more than or equal to 1 to 1 [mol/mol].

2. Composition according to claim 1, whereby the hydrogen peroxide-releasing substances are selected from a group **B21** consisting of
• hydrogen peroxide-urea adduct and
• sodium carbonate-hydrogen peroxide adduct
and the hydrogen peroxide-releasing mixtures of substances are selected from a group **B22** consisting of
• sodium carbonate-hydrogen peroxide adduct / 2-ethylhexanoic acid,
• calcium peroxide / 2-ethylhexanoic acid and
• magnesium peroxide / 2-ethylhexanoic acid.

3. Composition according to at least one of the preceding claims, whereby hydrogen peroxide is present in an amount of 20 ppm to 0.5 % by weight, relative to the monomer or mixtures of monomers.

4. Composition according to claim 3, whereby the amount is 20 ppm to 0.01 % by weight.

5. Composition according to at least one of the preceding claims, whereby the stabiliser C is a member of the group consisting of hydroquinone, hydroquinone monomethylether, alkylgallate, palmitoylascorbic acid, and 2,6-di-t-butyl-4-methylphenol.

6. Composition according to any one of the preceding claims, containing
**A1** a low-molecular liquid methacrylate;
**A2** a linear or branched polymethylmethacrylate or a linear or branched methylmethacrylate copolymer;
**A3** a cross-linked polymethylmethacrylate or a cross-linked methylmethacrylate copolymer;
**B1** hydrogen peroxide and/or
**B2** a hydrogen peroxide-releasing substance or a hydrogen peroxide-releasing mixture of substances; and
**C** at least one radical stabiliser;
**D** at least one component of a radical initiator system.

7. Use of a composition according to at least one of the claims 1-5 for producing
• one-component bone cement pastes,
• two-component bone cement pastes or
• monomer solutions for polymethylmethacrylate bone cements made of cement powder and monomer liquid.

## Revendications

1. Composition sporocide comprenant
**A** un monomère de méthacrylate ou un mélange de monomères de méthacrylate,
**B1** du péroxyde d'hydrogène et/ou
**B2** une substance libérant du péroxyde d'hydrogène ou un mélange de substances libérant du péroxyde d'hydrogène et
**C** au moins un stabilisateur radical,
sachant que la composante **B1** et le péroxyde d'hydrogène libéré de la composante **B2** ainsi que la composante C sont dissouts de façon homogène dans la composante **A**, et que le rapport de quantités de substances de **C** à **(B1+B2)** est supérieur ou égal à 1 à 1 [mol/mol].

2. Composition selon la revendication 1, dans laquelle les substances libérant du péroxyde d'hydrogène sont sélectionnées parmi un groupe B21 se composant de
• un produit d'addition péroxyde d'hydrogène-urée et
• un produit d'addition péroxyde d'hydrogène-carbonate de sodium
et les mélanges de substances libérant du péroxyde d'hydrogène sont sélectionnés parmi un groupe **B22** se composant de
• un produit d'addition péroxyde d'hydrogène-carbonate de sodium/2-acide éthylhexane,
• péroxyde de calcium/ 2-acide éthylhexane et
• péroxyde de magnésium/ 2-acide éthylhexane.

3. Composition selon l'une des revendications précédentes, dans laquelle le péroxyde d'hydrogène est présent dans une quantité de 20 ppm jusqu'à 0,5 % en poids, rapporté au monomère ou au mélange de monomères.

4. Composition selon la revendication 3, dans laquelle la quantité est de 20 ppm jusqu'à 0,01 % en poids.

5. Composition selon l'une des revendications précédentes, dans laquelle le stabilisateur C appartient au groupe composé de l'hydroquinone, de l'éther monométhylique d'hydroquinone, d'alkylgallate, d'acide ascorbique palmitoyle et de 2,6-di-t-butyl-4-méthylphénol.

6. Composition selon l'une des revendications précédentes, comprenant
**A1** un méthacrylate liquide de faible poids moléculaire,
**A2** un polyméthacrylate de méthyle linéaire ou ramifié ou un copolymère de méthylméthacrylate linéaire ou ramifié,
**A3** un polyméthylméthacrylate réticulé ou un copolymère de méthylméthacrylate réticulé,
**B1** du péroxyde d'hydrogène et/ou
**B2** une substance libérant du péroxyde d'hydrogène ou un mélange de substances libérant du péroxyde d'hydrogène et
C au moins un stabilisateur radical,
D au moins une composante d'un système d'amorçage radical.

7. Emploi d'une composition selon au moins l'une des revendications 1 - 5 pour la fabrication de
• pâtes de ciment osseux monocomposant,
• pâtes de ciment osseux bicomposants,
• solutions de monomères pour ciments osseux en polyméthylméthacrylate fabriqués en poudre de ciment et liquide monomère.
